# EUROPEAN PATENT SPECIFICATION

(11) **EP 2 123 271 B1**
(45) Date of publication and mention of the grant of the patent: **05.10.2011**
(21) Application number: 08721158.7
(22) Date of filing: 03.03.2008
(51) Int. Cl.: A61K 31/351, A61P 31/16, C07D 309/28

(54) **DRUG FOR TREATMENT OF INFLUENZA**
ARZNEIMITTEL ZUR BEHANDLUNG VON GRIPPE
MEDICAMENT DESTINE AU TRAITEMENT DE LA GRIPPE

(30) Priority: 07.03.2007 JP 2007056872
(43) Date of publication of application: 25.11.2009
(73) Proprietor: Daiichi Sankyo Company, Limited, Tokyo 103-0023 (JP)
(72) Inventor: YAMASHITA, Makoto, Tokyo 140-8710 (JP); KAWAOKA, Yoshihiro, Tokyo 113-0033 (JP)
(74) Representative: Wallace, Sheila Jane
(86) International application number: PCT/JP2008/053738
(87) International publication number: WO 2008/108323

(56) References cited:
- EP-A- 1 277 750
- JP-A- 10 330 373
- JP-A- 2002 012 590
- US-A1- 2002 137 791
- US-B1- 6 340 702
- LI J ET AL: "Syntheses of triazole-modified zanamivir analogues via click chemistry and anti-AIV activities" BIOORGANIC & MEDICINAL CHEMISTRY LETTERS, PERGAMON, ELSEVIER SCIENCE, GB, vol. 16, no. 19, 1 October 2006 (2006-10-01), pages 5009-5013, XP025107205 ISSN: 0960-894X [retrieved on 2006-10-01]
- MOSCONA A.: 'Oseltamivir resistance - disabling our influenza defenses' THE NEW ENGLAND JOURNAL OF MEDICINE vol. 353, no. 25, 2005, pages 2633 - 2636, XP008112475
- RUSSELL R.J. ET AL.: 'The structure of H5N1 avian influenza neuraminidase suggests new opportunities for drug design' NATURE vol. 443, 2006, pages 45 - 49, XP002474738
- LE Q.M. ET AL.: 'Avian flu: isolation of drug-resistant H5N1 virus' NATURE vol. 437, no. 7062, 2005, page 1108, XP008112478

## Description

### TECHNICAL FIELD

The present invention relates to a therapeutic or prophylactic for H5N1 influenza, comprising certain compounds represented by formula (I) as an active ingredient.

### BACKGROUND ART

Infection of poultry with H5N1 avian influenza virus has been expanding since 2003 in wide areas including Asia, Europe and Africa. Humans infected with this virus have been found not only in Asia but also in Middle East and Africa. If a new type of H5N1 influenza virus has appeared and its infection has started, it is believed that the infection will rapidly expand to cause a worldwide spread (i.e., influenza pandemic) because most people do not possess immunity against that virus and influenza viruses spread via droplet infection and airborne infection. More than half of human patients infected with H5N1 influenza virus have died so far. Thus, the virus is highly pathogenic. It is known that three influenza pandemics, the Spanish Flu, the Asian Flu and the Hong Kong Flu, occurred in the 20th century. In the Spanish Flu which caused the largest number of patients, it is estimated that about 20-40 million people died in the world and about 0.5 million people in Japan.

According to a report from Japanese Ministry of Health, Labour and Welfare made in November, 2005, if a new type influenza virus has spread, the number of patients who will consult medical doctors in Japan as a result of infection with that virus is estimated about 18-25 million. Further, when the pathogenicity of that new type influenza virus is severe, the number of inpatients is estimated about 0.2 million while the number of dead is estimated about 0.64 million. Therefore, not only health hazard but also significant influences upon social activities are feared.

Thus, a new type influenza can cause a highly severe disease. Early development of effective therapeutics is demanded.

Although it is reported that zanamivir (in particular, zanamivir hydrate) and oseltamivir (in particular, oseltamivir phosphate or oseltamivir carboxylate) which are influenza therapeutics with neuraminidase inhibitory activity show an inhibitory activity against H5N1 influenza virus, compounds with more excellent activity are desired (Non-Patent Document 1 or 2). Further, H5N1 influenza virus strains against which oseltamivir does not show any inhibitory activity (i.e., oseltamivir resistant virus strains) have been reported. Compounds which possess an inhibitory activity against these oseltamivir resistant H5N1 influenza virus strains are desired (Non-Patent Document 1 or 2).

Compounds represented by formula (I) are known to be useful as influenza therapeutics with neuraminidase inhibitory activity (Patent Documents 1 to 3). However, it has not been reported that these compounds have an inhibitory activity against H5N1 influenza virus. Further, the structures of the compounds represented by formula (I) resemble the structure of zanamivir but are completely different from the structure of oseltamivir.
Non-Patent Document 1: Nature, 2005, vol. 437, p. 1108
Non-Patent Document 2: N. Engl. J. Med., 2005, vol. 353, (25):2667-72
Patent Document 1: United States Patent No. 6340702 (Japanese Patent No. 3209946)
Patent Document 2: United States Patent No. 6451766 (Japanese Patent Publication No. Hei 10-109981)
Patent Document 3: United States Patent No. 6844363 (Japanese Patent Publication No. 2002-012590)

### DISCLOSURE OF THE INVENTION

### PROBLEM TO BE SOLVED BY THE INVENTION

The inventors have diligently investigated into therapeutics or prophylactics for influenza. The present inventors have found that certain compounds represented by the general formula (I) are extremely useful as therapeutics or prophylactics for H5N1 influenza. The present invention has been achieved based on this finding.

### MEANS TO SOLVE THE PROBLEM

The present invention provides the therapeutic or prophylactic for H5N1 influenza as disclosed below.
Compounds represented by the general formula (I) have the following structure: 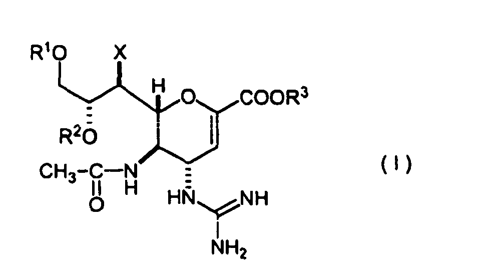 wherein R¹ and R² may be the same or different from each other and each represents a hydrogen atom or an alkanoyl group with 2 to 20 carbon atoms; X represents a halogen atom, a hydroxyl group, an alkoxy group with 1 to 4 carbon atoms or an alkanoyloxy group with 2 to 20 carbon atoms; and R³ represents a hydrogen atom or an alkyl group with 1 to 20 carbon atoms; PROVIDED THAT compounds of the general formula (I) wherein each of R¹ and R² is a hydrogen atom, X is a hydroxyl group, and R³ is a hydrogen atom are excluded.

According to the present invention there is provided:
(1) A compound selected from the group consisting of:
   5-acetamido-4-guanidino-9-O-hexanoyl-2,3,4,5-tetradeoxy-7-O-methyl-D-glycero-D-galacto-non-2- enopyranosoic acid,
   5-acetamido-4-guanidino-9-O-octanoyl-2,3,4,5-tetradeoxy-7-O-methyl-D-glycero-D-galacto-non-2- enopyranosoic acid,
   5-acetamido-4-guanidino-9-O-decanoyl-2,3,4,5-tetradeoxy-7-O-methyl-D-glycero-D-galacto-non-2- enopyranosoic acid,
   5-acetamido-4-guanidino-9-O-dodecanoyl-2,3,4,5-tetradeoxy-7-O-methyl-D-glycero-D-galacto-non-2- enopyranosoic acid, and
   5-acetamido-4-guanidino-9-O-tetradecanoyl-2,3,4,5-tetradeoxy-7-O-methyl-D-glycero-D-galacto-non-2- enopyranosoic acid,
   for use in the treatment or prevention of H5N1 influenza.
(2) Use of a compound selected from the group consisting of:
   5-acetamido-4-guanidino-9-O-hexanoyl-2,3,4,5-tetradeoxy-7-O-methyl-D-glycero-D-galacto-non-2- enopyranosoic acid,
   5-acetamido-4-guanidino-9-O-octanoyl-2,3,4,5-tetradeoxy-7-O-methyl-D-glycero-D-galacto-non-2- enopyranosoic acid,
   5-acetamido-4-guanidino-9-O-decanoyl-2,3,4,5-tetradeoxy-7-O-methyl-D-glycero-D-galacto-non-2- enopyranosoic acid,
   5-acetamido-4-guanidino-9-O-dodecanoyl-2,3,4,5-tetradeoxy-7-O-methyl-D-glycero-D-galacto-non-2- enopyranosoic acid, and
   5-acetamido-4-guanidino-9-O-tetradecanoyl-2,3,4,5-tetradeoxy-7-O-methyl-D-glycero-D-galacto-non-2- enopyranosoic acid, for the preparation of a medicament for the prevention or treatment of H5N1 influenza.

In the above disclosure, the compounds represented by the general formula (I) may be exemplified by, the 2-deoxy-2,3-didehydro-N-acetylneuraminic acid derivatives described in United States Patent No. 6340702 (Japanese Patent No. 3209946), United States Patent No. 6451766 (Japanese Unexamined Patent Publication No. Hei 10-109981), United States Patent No. 6844363 (Japanese Unexamined Patent Publication No. 2002-012590), and so forth.

In the compounds claimed in claim 1 and represented by the general formula (I), when R¹ or R² is an alkanoyl group, when X is an alkanoyloxy group or when R³ is an alkyl group, R¹O-, R²O-, X or R³OCO individually forms an ester group. When the compound claimed in claim 1 possesses these ester groups, the compound can be a prodrug (Development of Pharmaceuticals published by Hirokawa Shoten, 1990, vol. 7, "Molecular Design", pp. 163-198). Once administered, the above-described ester group in the compound claimed in claim 1 is converted to a hydroxyl or carboxyl group through *in vivo* metabolic processes (e.g., hydrolysis), and the compound produced by this conversion exhibits a pharmacological activity (see, for example, Test Examples 1' to 4' in United States Patent No. 6451766 and Test Examples 1 to 4 in Japanese Patent Publication No. Hei 10-109981).

Since the compounds claimed in claim 1 possess a guanidino group or a carboxyl group in their molecules, the compounds are capable of forming a pharmacologically acceptable salt by binding to a pharmacologically non-toxic acid or base.

Examples of the "pharmacologically acceptable salt" include hydrohalogenic acid salts such as hydrofluoride, hydrochloride, hydrobromide, and hydroiodide; inorganic acid salts such as nitrate, perchlorate, sulfate, and phosphate; alkanesulfonates such as methanesulfonate, ethanesulfonate, and trifluoromethanesulfonate; arylsulfonates such as benzenesulfonate, and p-toluenesulfonate; organic acid salts such as acetate, trifluoroacetate, citrate, tartrate, oxalate, and maleate; amino acid salts such as glycine salt, lysine salt, arginine salt, ornithine salt, glutamic acid salt, and aspartic acid salt; alkali metal salts such as lithium salt, sodium salt, and potassium salt; alkaline earth metal salts such as calcium salt, and magnesium salt; metal salts such as aluminum salt, iron salt, zinc salt, copper salt, nickel salt, and cobalt salt; organic amine salts or organic ammonium salts such as ammonium salt, t-octylamine salt, dibenzylamine salt, morpholine salt, glucosamine salt, ethylenediamine salt, guanidine salt, diethylamine salt, triethylamine salt, dicyclohexylamine salt, procaine salt, ethanolamine salt, diethanolamine salt, piperazine salt, and or tetramethylammonium salt. Preferably, the pharmacologically acceptable salt is an alkali metal salt, an organic acid salt or an inorganic acid salt.

The compounds claimed in claim 1 may form hydrates or solvates when left in the air or mixed with water or an organic solvent.

The above-described pharmacologically acceptable salts, hydrates or solvates of the compounds claimed in claim 1 are included in the scope of the active ingredient of the therapeutic or prophylactic of the present invention.

The compounds claimed in claim 1 are extremely useful as therapeutics or prophylactics for H5N1 influenza. The target H5N1 influenza may be an influenza caused by an oseltamivir sensitive or resistant H5N1 influenza virus. Preferably, the target H5N1 influenza is an influenza caused by an oseltamivir resistant H5N1 influenza virus. The oseltamivir sensitive H5N1 influenza virus may be, for example, A/Hanoi/30408/2005 (clone 7), while the oseltamivir resistant H5N1 influenza virus may be, for example, A/Hanoi/30408/2005 (clone 9). From another viewpoint, the compounds claimed in claim 1 are also useful as therapeutics or prophylactics for influenza caused by oseltamivir resistant influenza viruses (not limited to H5N1 viruses).

### EFFECT OF THE INVENTION

The compounds claimed in claim 1 are extremely useful as therapeutics or prophylactics for H5N1 influenza, and also useful as therapeutics or prophylactics for influenza caused by oseltamivir resistant H5N1 influenza viruses.

The present specification encompasses the contents of the specification and/or the drawings of Japanese Patent Application No. 2007-56872 based on which the present patent application claims priority.

### BEST MODE FOR CARRYING OUT THE INVENTION

The compounds claimed in claim 1 which are the active ingredients of the therapeutics or prophylactics of the present invention may be prepared according to the methods disclosed in United States Patent No. 6340702 (Japanese Patent No. 3209946), United States Patent No. 6451766 (Japanese Patent Publication No. Hei 10-109981), United States Patent No. 6844363 (Japanese Patent Publication No. 2002-012590), etc. or the methods equivalent to those methods.

When the compounds claimed in claim 1 are used as therapeutics or prophylactics for influenza, the compounds may be administered (i) as they are, or (ii) in the form of a preparation formulated by mixing with appropriate pharmacologically acceptable excipients, diluents or the like (e.g., tablet, capsule, granule, powder, syrup, ointment, liquid, suspension, aerosol or troche).

These preparations may be prepared by well-known methods using additives such as excipients, binders, disintegrating agents, lubricants, stabilizers, corrigents, suspending agents, diluents and solvents for preparations.

Examples of the excipient include: saccharides such as lactose, sucrose, glucose, mannitol, and sorbitol; starch derivatives such as corn starch, potato starch, α-starch, dextrin and carboxymethyl starch; cellulose derivatives such as crystalline cellulose, low-substituted hydroxypropyl cellulose, hydroxypropylmethyl cellulose, carboxymethyl cellulose, carboxymethyl cellulose calcium, and internally crosslinked sodium carboxymethyl cellulose; gum arabic; dextran; pullulan; silicates such as light silicic acid anhydride, synthetic aluminum silicate and magnesium aluminometasilicate; phosphates such as calcium phosphate; carbonates such as calcium carbonate; and sulfates such as calcium sulfate.

Examples of the binder include the excipients listed above; gelatin; polyvinylpyrrolidone; and macrogol.

Examples of the disintegrating agent include: the excipients listed above; and chemically modified starch or cellulose derivatives such as sodium cross-carmellose, sodium carboxymethyl starch, and crosslinked polyvinylpyrrolidone.

Examples of the lubricant include: talc; stearic acid; metal salts of stearic acid such as calcium stearate and magnesium stearate; colloidal silica; veegum; waxes such as beeswax and spermaceti; boric acid; glycol; carboxylic acids such as fumaric acid or adipic acid; sodium carboxylates such as sodium benzoate; sulfates such as sodium sulfate; leucine; lauryl sulfates such as sodium lauryl sulfate and magnesium lauryl sulfate; silicic acids such as silicic acid anhydride and silicic acid hydrate; and the starch drivatives listed above in as examples of excipients.

Examples of the stabilizer include: para-hydroxybenzoic acid esters such as methylparaben and propylparaben; alcohols such as chlorobutanol, benzyl alcohol and phenethyl alcohol; benzalkonium chloride; phenols such as phenol and cresol; thimerosal; acetic anhydride; and sorbic acid.

The corrigent may be, for example, conventionally used sweeteners, acidifiers, or flavors.

The suspending agent may be, for example, polysorbate 80 or sodium carboxymethyl cellulose.

The solvent for preparations may be, for example, water, ethanol, or glycerol.

The therapeutic or prophylactic of the present invention may be administered orally or parenterally. Preferably, the therapeutic or prophylactic is administered by an administration method that allows delivery of the active ingredient to the respiratory organ tissue (tissue of the oral cavity, nasal cavity, respiratory tracts or lungs tissue) of the recipient which is the major route of infection with influenza virus. Administration methods may be, for example, nasal drop, transnasal, transpulmonary or intraoral administration. Generally, the active ingredient may be administered in the form of a solution, suspension or dry powder. Solutions and suspensions are aqueous and may be prepared generally with water (e.g., sterile water or pyrogen-free water) alone or with water and a pharmacologically acceptable auxiliary solvent (e.g., ethanol, propylene glycol, polyethylene glycol or PEG 400). Such solutions or suspensions may further contain other excipients, antiseptics (e.g., benzalkonium chloride), solubilizing agents/surfactants such as polysorbate (e.g., Tween 80, Span 80 or benzalkonium chloride), buffers, isotonicity regulators (e.g., sodium chloride), absorption enhancers or thickening agents. Suspensions may further contain suspending agents (e.g., microcrystalline cellulose or sodium carboxymethyl cellulose). Solutions and suspensions are directly administered into the nasal cavity or oral cavity by conventional means (such as dropper, pipette or sprayer). Solutions and suspensions may be supplied in a single or multiple dosage form. In the latter case, it is preferred that a means to measure the dose be provided. When a dropper or pipette is used for administration, the patient administers an appropriate specific volume of the solution or suspension to himself/herself. When a sprayer is used, the solution or suspension may be administered by means of a measuring spray pump, for example. Administration to the respiratory tracts or lungs may be achieved by using an aerosol formulation in the form of a pressurized pack composed of the active ingredient and an appropriate propellant [e.g., gas such as chlorofluorocarbon (CFC), dichlorodifluoromethane, trichlorofluoromethane, dichlorotetrafluoroethane or carbon dioxide]. Preferably, the aerosol formulation contains a surfactant such as lecitin. Dose levels of the active ingredient may be regulated by providing the relevant administration instrument with a measuring valve. Further, the active ingredient may be provided as a dry powder of itself. Alternatively, the active ingredient may be provided in the form of a powder mixture obtained by mixing the active ingredient with an appropriate powder base such as lactose, starch, starch derivative (e.g., hydroxypropyl methyl cellulose) or polyvinylpyrolidone (PVP). It is preferred that the above-described dry powder or powder mixture form a gel in the nasal cavity. The above-described dry powder or powder mixture may be supplied in a unit dosage form using, for example, capsules or cartridges made from gelatin or blister packs, and administered from these capsules, cartridges or blister packs with an inhaler. In formulations to be administered to the respiratory tracts or lungs (including compositions for intranasal administration), the particle size of the active ingredient is generally small. The active ingredient with such a small particle size may be obtained by a method such as submicronizing which is well-known in the field of drug formulation. It is also possible to use a formulation that is designed to release the active ingredient in a sustained manner.

The therapeutic or prophylactic of the present invention is most preferably administered as a powder mixture by inhalation through the nose or mouth.

The therapeutic or prophylactic of the present invention may be used in combination with other therapeutics. Preferable examples of other therapeutics which may be used include influenza therapeutics such as oseltamivir (in particular, oseltamivir phosphate or oseltamivir carboxylate), zanamivir (in particular, zanamivir hydrate), amantadine (in particular, amantadine hydrochloride), rimantadine and ribavirin. When used in such combinations, the individual active ingredients may be administered successively or simultaneously either as separate pharmaceutical formulations or as a single pharmaceutical composition containing all the active ingredients. When the therapeutic or prophylactic of the present invention is used in combination with other therapeutics which are active against the same target virus, the dose levels of the respective active ingredient may be the same as or different from those employed when they are used alone.

The administration of the therapeutic or prophylactic of the present invention is initiated depending on the need when a pandemic has occurred or when a person is going to an area where influenza is spreading in poultry. The administration may be performed 1 to 7 times per week, the frequency being increased or decreased depending on the need. When a pandemic has occurred, it is possible to increase the number of doses or start the administration in advance, if viral spread is imminent, if a person is living in a group, if a person is living or working in a place where many and unspecified people gather (e.g., day nurseries, kindergartens, schools, companies/firms, hospitals, homes for the aged, movie theaters, libraries, concert halls or places to sports stadiums) or if a person is going to visit such a place. Even when a person is going to an area where influenza is spreading in poultry for the purpose of investigation, traveling or the like, it is possible to increase the number of doses or to start the administration in advance. "Increasing the number of doses" means administering, for example, once a day. "Administering in advance" means administering before a person takes an action that may result in influenza infection or administering after that action but before the onset of influenza.

While the dose level of the therapeutic or prophylactic of the present invention will vary depending on the type of active ingredient used, the extent of spread of influenza and conditions of the patient (body weight, age, etc.), if it is to be administered to human by inhalation, it is desirable to administer the active ingredient at a dose of 10 µg to 5 mg per kg of body weight about once to 7 times a week to once to 3 times a day.

The active ingredient of the therapeutic or prophylactic of the present invention is capable of inhibiting neuraminidase which is essential for the proliferation of H5N1 influenza virus to thereby inhibit the proliferation of this virus. This neuraminidase inhibitory activity may be evaluated, for example, by the methods described below. However, the evaluation method is not limited to the following methods.

For example, it is possible to quantitatively evaluate the neuraminidase inhibitory activity of the active ingredient of the therapeutic or prophylactic of the present invention by mixing H5N1 influenza virus having neuraminidase enzyme activity with a substrate for neuraminidase to thereby prepare a reaction system for enzyme activity detection, and adding the active ingredient of the therapeutic or prophylactic of the present invention to this system.

Alternatively, it is also possible to quantitatively evaluate the H5N1 influenza virus proliferation inhibiting activity of the active ingredient of the therapeutic or prophylactic of the present invention by infecting cultured cells with H5N1 influenza virus to thereby prepare an experimental system for plaque formation based on viral proliferation, adding the active ingredient to the system, and measuring a decrease in plaque number or size or the amount of virus in the culture broth.

The therapeutic or prophylactic effect on influenza virus infection as offered by the therapeutic or prophylactic of the present invention may be evaluated by the methods described below. However, the evaluation method is not limited to the following methods.

For example, an appropriate amount of the active ingredient of the therapeutic or prophylactic of the present invention is administered in the form of a solution, suspension or powder to the respiratory organ of a vertebrate (such as human, mouse, rat, ferret, pig or bird) by an administration method such as nasal drop, intranasal, intrataracheal or intratpulmonary administration or inhalation. At an appropriate time between immediately after the administration and one month after the administration, influenza virus is inhaled or added dropwise to the nose once so that the vertebrate becomes infected. Subsequently, any symptoms of influenza (e.g., fever; headache; general malaise; joint pain; muscular pain; respiratory organ symptoms such as cough or sputum; the amount of virus contained in the fluid on throat swab, nasal discharge or lung lavage fluid; survival or death, etc.) are observed or measured. Thus, it is possible to evaluate the therapeutic or prophylactic effect of interest.

Alternatively, a group of humans is prepared to whom an appropriate amount of the active ingredient of the therapeutic or prophylactic of the present invention is administered to the respiratory tracts (including passage through the nose) by oral, rectal, nasal, local (including intraoral and sublingual), vaginal, parenteral (including intramuscular, subcutaneous and intravenous) administration or inhalation in an area where influenza is spreading. On the other hand, another group of humans is prepared to whom the active ingredient of the therapeutic or prophylactic of the present invention is not administered. After a specific time period, the proportions of persons who were infected with influenza virus to develop any symptoms of influenza in both groups are statistically examined. Thus, it is possible to evaluate the therapeutic or prophylactic effect of interest.

To evaluate the prophylactic effect in mice, the active ingredient of the therapeutic or prophylactic of the present invention as dissolved in physiological saline or an appropriate buffer is intranasally administered by adding a suitable amount of the solution dropwise into the nasal cavity. The mice are intranasally infected with influenza virus in the same manner at an appropriate time between immediately after the administration and one month after the administration. After the infection, the lungs are removed from each mouse, followed by measurement of the virus titers in the lungs. Thus, the prophylactic effect can be evaluated. If the influenza virus used causes lethal infection in mice, the prophylactic effect can be evaluated by observing the survival and death of the mice.

### EXAMPLES

Hereinbelow, the present invention will be described more specifically with reference to the following Preparation Examples, Examples and Formulation Examples. However, the scope of the present invention is not limited to these Examples.

### PREPARATION EXAMPLE 1

### 5-Acetamido-4-guanidino-9-O-octanoyl-2-,3,4,5-tetradeoxy-7-O-methyl-D-glycero-D-galacto-non-2-enopyranosoic acid

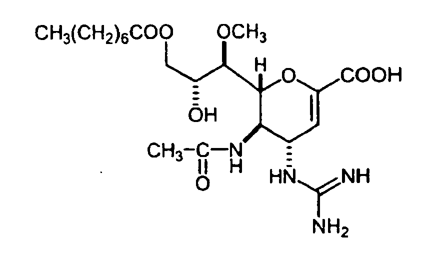

(1) Diphenylmethyl 5-acetamido-4-(N,N'-bis-t-butyloxycarbonyl)guanidino-9-O-octanoyl-2,3,4,5-tetradeoxy-7-O-methyl-D-glycero-D-galacto-non-2-enopyranosoate (3.46 g, 4.1 mmol) disclosed in Example 35 (i) of United States Patent No. 6340702 (Japanese Patent No. 3209946) was dissolved in methylene chloride (27 ml) and trifluoroacetic acid (14 ml). The resultant solution was stirred at room temperature overnight. The reaction solution was concentrated to dryness under reduced pressure, followed by three cycles of azeotropic distillation to dryness with toluene (5 ml). The resultant oily material was dissolved in ethyl acetate (10 ml). The solution was poured into a saturated aqueous solution of sodium hydrogencarbonate (50 ml). The pH of the resultant solution was adjusted to 8.5 by addition of 20% aqueous solution of sodium carbonate. Then, the solution was stirred at room temperature for 3 hr and its pH was adjusted to 5.0 with hydrochloric acid (3 ml), followed by stirring at room temperature for another 1 hr. The solution was further stirred for 1 hr while ice-cooling. Subsequently, precipitating crystals were suction filtered and vacuum dried for 10 hr at an external temperature of 50°C. The resultant crystals were left in the air for one day to thereby yield the subject compound as a hydrate crystal (0.97 g; yield 51%).
   Infrared Absorption Spectrum (KBr) ν max cm⁻¹: 3412, 2929, 2856, 1676, 1401, 1320, 1285, 1205, 1137, 722.
   ¹H Nuclear Magnetic Resonance Spectrum (400MHz, CD₃OD) δ ppm: 5.88 (1H, d, J=2.5 Hz), 4.45 (3H, m), 4.27 (1H, dd, J=10.0 Hz, 10.0 Hz), 4.15 (1H, m), 3.47 (2H, m), 3.42 (3H, s), 2.37 (2H, t, J=7.4 Hz), 2.10 (3H, s), 1.31 (2H, m), 1.20-1.40 (8H, m), 0.85-0.95 (3H, m).
   ¹³C Nuclear Magnetic Resonance Spectrum (100MHz, CD₃OD) δ ppm: 176.5, 173.7, 164.7, 158.9, 146.7, 108.7, 80.1, 78.0, 69.3, 66.8, 61.4, 52.4, 35.1, 32.8, 30.2, 30.1, 26.0, 23.7, 22.8, 14.4.
(2) The subject compound was also obtained by the method described below.
   5-Acetamido-4-guanidino-9-O-octanoyl-2,3,4,5-tetradeoxy-7-O-methyl-D-glycero-D-galacto-non-2-enopyranosoic acid trifluoroacetic acid salt (3.0 g, 5.1 mmol) disclosed in Example 35 (ii) of United States Patent No. 6340702 (Japanese Patent No. 3209946) was subjected to reversed phase column chromatography [Cosmosil 75C 18PREP (nacalai tesque), 100 g] and eluted with methanol/water (0/1-1/1-2/1). Fractions containing the compound of interest were vacuum concentrated. The precipitating crystals were suction filtered and vacuum dried. The resultant crystals were left in the air for one day to thereby yield the subject compound as a hydrate crystal (1.2 g; yield 49%). The property data of the resultant compound were consistent with those of the compound obtained in (1) above.

### PREPARATION EXAMPLE 2

### 5-Acetamido-4-guanidino-2,3,4,5-tetradeoxy-7-O-methyl-D-glycero-D-galacto-non-2-enopyranosoic acid

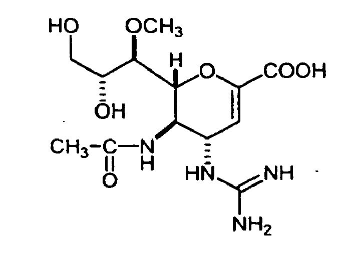

5-Acetamido-4-guanidino-2,3,4,5-tetradeoxy-7-O-methyl-D-glycero-D-galactonon-2-enopyranosoic acid trifluoroacetic acid salt (3.0 g, 5.1 mmol) disclosed in Example 28 (viii) of United States Patent No. 6340702 (Japanese Patent No. 3209946) was purified in an ion-exchange resin column [Dowex-50X; (i) water and (ii) 5% aqueous ammonium solution] and further purified by reversed phase column chromatography [Cosmosil 75C 18PREP (nacalai tesque); water]. Fractions containing the compound of interest were vacuum concentrated. The resultant solid was washed with methanol, filtered and dried to thereby yield the subject compound (1.43 g) as a colorless solid.
¹H Nuclear Magnetic Resonance Spectrum (400MHz, CD₃OD) δ ppm: 5.64 (1H, d, J=2.0 Hz), 4.43 (2H, m), 4.22 (1H, dd, J=10.0 Hz, 10.0 Hz), 4.00 (1H, m), 3.85 (1H, dd, J = 10.0 Hz, 2.4 Hz), 3.68 (1H, dd, J= 10.0 Hz, 5.5 Hz), 3.58 (1H, m), 3.43 (3H, s).
Note that the compound of preparation Example 2 is the one that derives from the compound of Preparation Example 1 when it is metabolized *in vivo.* In other words, the compound of Preparation Example 1 is a prodrug of the compound of Preparation Example 2 whereas the compound of Preparation Example 1 is the active form per se.

### EXAMPLE 1 Neuraminidase Inhibitory Activity Test

The following test was performed according to the method disclosed in Nature, 2005, vol. 437, p. 1108 with necessary modifications.

An H5N1 virus solution diluted to give a neuraminidase activity of 800 to 1200 fluorescent units was mixed with a test compound adjusted at 0.01 to 100000 nM. The mixture was reacted at 37°C for 30 min. Subsequently, 0.1 mM 2'-(4-methylumbelliferyl)-α-D-N-acetylneuraminic acid as a substrate was added to the reaction system, which was further reacted at 37°C for 1 hr. Then, measurements were conducted at an excitation wavelength of 360 nm and an emission wavelength of 465 nm. From the resultant fluorescence intensity, inhibition rates (%) of the test compound at various concentrations were calculated with the neuraminidase activity in the absence of the test compound taken as 100. The concentration of the test compound giving 50% inhibition of the enzyme activity (IC₅₀) was calculated. The results are shown in Table 1.

Possessing a potent neuraminidase inhibitory activity against oseltamivir sensitive and resistant H5N1 influenza virus strains, the active ingredient of the therapeutic or prophylactic of the present invention exhibited an excellent inhibitory activity even against oseltamivir resistant H5N1 influenza virus strain.

**Table 1. Neuraminidase Inhibitory Activity (IC₅₀, nM)**

| | Oseltamivir sensitive strain | Oseltamivir resistant strain |
|---|---|---|
| Compound of Preparation Example 2 | 0.33 | 1.3 |
| Zanamivir | 0.96 | 0.81 |
| Oseltamivir carboxylate | 0.34 | 550 |

| | | |
|---|---|---|
| Oseltamivir sensitive strain: A/Hanoi/30408/2005 (clone 7) Oseltamivir resistant strain: A/Hanoi/30408/2005 (clone 9) | | |

### EXAMPLE2 Viral Proliferation Inhibitory Activity Test

H5N1 influenza virus was inoculated into MDCK cells so that 50 to 100 plaques would be formed per well. The virus was adsorbed onto the cells at 37°C for 1hr. Then, an agar medium containing a test compound at concentrations of 0.1 to 1000 nM was overlayed, and the MDCK cells were cultured for 2 days. Subsequently, the resultant culture was fixed and stained with 0.1% Crystal Violet dissolved in 19% methanol, and then the number and diameter of plaques were measured. In order to calculate inhibition rate based on the plaque area, the sum of the square of each plaque diameter was calculated. The inhibition rate (%) of the test compound at various concentrations was calculated with the inhibition rate in the absence of the test compound taken as 100%. Then, the concentration of the test compound giving 50% inhibition of viral proliferation (IC₅₀) was calculated. The results are shown in Table 2.

Possessing a potent viral proliferation inhibitory activity against oseltamivir sensitive and resistant H5N1 influenza virus strains, the active ingredient of the therapeutic or prophylactic of the present invention exhibited a quite outstanding viral proliferation inhibitory activity compared to zanamivir.

**Table 2. Viral Proliferation Inhibitory Activity (IC₅₀, nM)**

| | Oseltamivir sensitive strain | Oseltamivir resistant strain |
|---|---|---|
| Compound of Preparation Example 2 | 0.43 | 13 |
| Zanamivir | 3.3 | 32 |
| Oseltamivir carboxylate | 0.11 | 1400 |

| | | |
|---|---|---|
| Oseltamivir sensitive strain: A/Hanoi/30408/2005 (clone 7) Oseltamivir resistant strain: A/Hanoi/30408/2005 (clone 9) | | |

### EXAMPLE 3 Test of Treatment of Infected Mouse 1

Physiological saline alone or a test compound dissolved in physiological saline at various concentrations is administered to Balb/c mice intranasally. Seven days after the administration, H5N1 influenza virus is intranasally inoculated into the mice for infection. On day 1, day 2 and day 3 after the infection, the lungs are removed from the mice, and H5N1 influenza virus contained therein is quantitatively determined. Thus, the proliferation inhibitory effect of the test compound on H5N1 influenza virus is evaluated.

The mice administered with the active ingredient of the therapeutic or prophylactic of the present invention exhibit a significant decrease in the amount of virus contained in the lung, as compared to the mice administered with physiological saline alone.

### EXAMPLE 4 Test of Treatment of Infected Mouse 2

Test compounds are administered to mice in the same manner as in Example 3. Then, the survival of the mice inoculated with H5N1 influenza virus is observed until 20 days after the infection.

Almost all mice administered with physiological saline alone die in 20 days after the infection, but some of the mice administered with the active ingredient of the therapeutic or prophylactic of the present invention are still alive even at 20 days after the infection.

Specifically, 50 µl of a solution containing 80 pfu (plaque forming units) of A/Hanoi/UT30408(clone 7)/2005 (H5N1) isolated from patients infected with H5N1 avian influenza virus was administered dropwise to mice (Balb/c, female, 6-week old) intranasally. The compound of Preparation Example 1 was dissolved in physiological saline, and 3% Tamiflu Dry Syrup^{™} (oseltamivir phosphate) was dissolved in distilled water. The concentration of the compound of Preparation Example 1 was adjusted to give a dose of 70 or 700 µg/kg/50 µl. The concentration of oseltamivir phosphate was adjusted to give a dose of 0.5, 5 or 50 mg/kg/200 µl. The compound of Preparation Example 1 was administered intranasally once 2 hours after the infection. Oseltamivir phosphate was administered orally once 2 hours after the infection, and twice per day for the following 5 days in a total of 10 times. The results are shown in terms of the number of mice surviving on day 6, day 8, day 10, day 15 and day 20 after the infection. Each test group consisted of 10 mice.

**Table 3.**

| | Day 10 | Day 15 | Day 20 |
|---|---|---|---|
| Physiological saline alone | 0 | 0 | 0 |
| Compound of Preparation Example 1 (70 µg/kg) | 7 | 3 | 2 |
| Compound of Preparation Example 1 (700 µg/kg) | 7 | 7 | 6 |
| Oseltamivir phosphate (0.5 mg/kg) | 4 | 3 | 2 |
| Oseltamivir phosphate (5 mg/kg) | 6 | 3 | 2 |
| Oseltamivir phosphate (50 mg/kg) | 9 | 8 | 8 |

All publications, patents and patent applications cited herein are incorporated herein by reference in their entirety.

### INDUSTRIAL APPLICABILITY

The compounds claimed in claim 1 are extremely useful as therapeutics or prophylactics for H5N1 influenza. The compounds are also useful as therapeutics or prophylactics for the influenza caused by oseltamivir resistant H5N1 influenza virus.

## Claims

1. A compound selected from the group consisting of:
5-acetamido-4-guanidino-9-O-hexanoyl-2,3,4,5-tetradeoxy-7-O-methyl-D-glycero-D-galacto-non-2- enopyranosoic acid,
5-acetamido-4-guanidino-9-O-octanoyl-2,3,4,5-tetradeoxy-7-O-methyl-D-glycero-D-galacto-non-2-enopyranosoic acid,
5-acetamido-4-guanidino-9-O-decanoyl-2,3,4,5-tetradeoxy-7-O-methyl-D-glycero-D-galacto-non-2- enopyranosoic acid,
5-acetamido-4-guanidino-9-O-dodecanoyl-2,3,4,5-tetradeoxy-7-O-methyl-D-glycero-D-galacto-non-2-enopyranosoic acid, and
5-acetamido-4-guanidino-9-O-tetradecanoyl-2,3,4,5-tetradeoxy-7-O-methyl-D-glycero-D-galacto-non-2- enopyranosoic acid,
for use in the treatment or prevention of H5N1 influenza.

2. Use of a compound selected from the group consisting of:
5-acetamido-4-guanidino-9-O-hexanoyl-2,3,4,5-tetradeoxy-7-O-methyl-D-glycero-D-galacto-non-2- enopyranosoic acid,
5-acetamido-4-guanidino-9-O-octanoyl-2,3,4,5-tetradeoxy-7-O-methyl-D-glycero-D-galacto-non-2- enopyranosoic acid,
5-acetamido-4-guanidino-9-O-decanoyl-2,3,4,5-tetradeoxy-7-O-methyl-D-glycero-D-galacto-non-2- enopyranosoic acid,
5-acetamido-4-guanidino-9-O-dodecanoyl-2,3,4,5-tetradeoxy-7-O-methyl-D-glycero-D-galacto-non-2- enopyranosoic acid, and
5-acetamido-4-guanidino-9-O-tetradecanoyl-2,3,4,5-tetradeoxy-7-O-methyl-D-glycero-D-galacto-non-2- enopyranosoic acid,
for the preparation of a medicament for the prevention or treatment of H5N1 influenza.

## Patentansprüche

1. Verbindung ausgewählt aus der Gruppe bestehend aus :
5-acetamido-4-guanidino-9-O-hexanoyl-2,3,4,5-tetradesoxy-7-O-methyl-D-glycero-D-galacto-non-2-enopyranosonsäure ;
5-acetamido-4-guanidino-9-O-octanoyl-2,3,4,5-tetradesoxy-7-O-methyl-D-glycero-D-galacto-non-2- enopyranosonsäure ;
5-acetamido-4-guanidino-9-O-decanoyl-2,3,4,5-tetradesoxy-7-O-methyl-D-glycero-D-galacto-non-2- enopyranosonsäure ;
5-acetamido-4-guanidino-9-O-dodecanoyl-2,3,4,5-tetradesoxy-7-O-methyl-D-glycero-D-galacto-non-2- enopyranosonsäure ; und
5-acetamido-4-guanidino-9-O-tetradecanoyl-2,3,4,5-tetradesoxy-7-O-methyl-D-glycero-D-galacto-non-2- enopyranosonsäure ;
zur Verwendung in der Behandlung oder Vorbeugung gegen die H5N1-Influenza.

2. Verwendung einer Verbindung ausgewählt aus der Gruppe bestehend aus:
5-acetamido-4-guanidino-9-O-hexanoyl-2,3,4,5-tetradesoxy-7-O-methyl-D-glycero-D-galacto-non-2-enopyranosonsäure ;
5-acetamido-4-guanidino-9-O-octanoyl-2,3,4,5-tetradesoxy-7-O-methyl-D-glycero-D-galacto-non-2- enopyranosonsäure ;
5-acetamido-4-guanidino-9-O-decanoyl-2,3,4,5-tetradesoxy-7-O-methyl-D-glycero-D-galacto-non-2- enopyranosonsäure ;
5-acetamido-4-guanidino-9-O-dodecanoyl-2,3,4,5-tetradesoxy-7-O-methyl-D-glycero-D-galacto-non-2- enopyranosonsäure ; und
5-acetamido-4-guanidino-9-O-tetradecanoyl-2,3,4,5-tetradesoxy-7-O-methyl-D-glycero-D-galacto-non-2- enopyranosonsäure ;
zur Herstellung eines Medikamentes für die Vorbeugung oder Behandlung der H5N1-Influenza.

## Revendications

1. Composé sélectionné dans le groupe constitué de :
acide 5-acétamido-4-guanidino-9-O-hexanoyl-2,3,4,5-tétradésoxy-7-O-méthyl-D-glycéro-D-galacto-non-2-énopyranosoïque ;
acide 5-acétamido-4-guanidino-9-O-octanoyl-2,3,4,5-tétradesoxy-7-O-méthyl-D-glycéro-D-galacto-non-2- énopyranosoïque ;
acide 5-acétamido-4-guanidino-9-O-décanoyl-2,3,4,5-tétradesoxy-7-O-méthyl-D-glycéro-D-galacto-non-2- énopyranosoïque ;
acide 5-acétamido-4-guanidino-9-O-dodécanoyl-2,3,4,5-tétradésoxy-7-O-méthyl-D-glycéro-D-galacto-non-2- énopyranosoïque ; et
acide 5-acétamido-4-guanidino-9-O-tétradécanoyl-2,3,4,5-tétradésoxy-7-O-méthyl-D-glycéro-D-galacto-non-2- énopyranosoïque ;
pour une utilisation dans le traitement ou la prévention de la grippe H5N1.

2. Utilisation d'un composé sélectionné dans le groupe constitué de:
acide 5-acétamido-4-guanidino-9-O-hexanoyl-2,3,4,5-tetradésoxy-7-O-méthyl-D-glycéro-D-galacto-non-2-énopyranosoïque ;
acide 5-acétamido-4-guanidino-9-O-octanoyl-2,3,4,5-tétradésoxy-7-O-méthyl-D-glycéro-D-galacto-non-2- énopyranosoïque ;
acide 5-acétamido-4-guanidino-9-O-décanoyl-2,3,4,5-tétradesoxy-7-O-méthyl-D-glycéro-D-galacto-non-2- énopyranosoïque ;
acide 5-acétamido-4-guanidino-9-O-dodécanoyl-2,3,4,5-tetradésoxy-7-O-méthyl-D-glycéro-D-galacto-non-2- énopyranosoïque ; et
acide 5-acétamido-4-guanidino-9-O-tétradecanoyl-2,3,4,5-tétradésoxy-7-O-méthyl-D-glycéro-D-galacto-non-2- énopyranosoïque ;
pour la fabrication d'un médicament pour la prévention ou le traitement de la grippe H5N1.
